# EUROPEAN PATENT SPECIFICATION

(11) **EP 4 288 383 B1**
(45) Date of publication and mention of the grant of the patent: **11.09.2024**
(21) Application number: 22702671.3
(22) Date of filing: 26.01.2022
(51) Int. Cl.: C01B 3/32, C01B 3/12, C01B 3/38, C01B 3/48, C01B 3/50, C01B 3/56, C07C 29/151

(54) **PROCESS AND PLANT FOR METHANOL PRODUCTION**
VERFAHREN UND ANLAGE ZUR METHANOLHERSTELLUNG
PROCÉDÉ ET INSTALLATION DE PRODUCTION DE MÉTHANOL

(30) Priority: 04.02.2021 EP 21155137
(43) Date of publication of application: 13.12.2023
(73) Proprietor: CASALE SA, 6900 Lugano (CH)
(72) Inventor: COLOMBO, Gabriele, 6963 Pregassona (CH); OSTUNI, Raffaele, 6900 Lugano (CH); IOCCHI, Filippo, 6900 Lugano (CH); FILIPPI, Ermanno, 6976 Castagnola (CH)
(74) Representative: M. Zardi & Co S.A.
(86) International application number: PCT/EP2022/051764
(87) International publication number: WO 2022/167288

(56) References cited:
- GB-A- 2 585 477
- US-A1- 2018 258 019
- US-B2- 6 706 770

## Description

### Field of application

The present invention relates to a process and plant for the synthesis of methanol.

### Prior art

The industrial preparation of methanol includes syngas preparation, methanol synthesis and methanol purification.

Syngas is prepared in a front-end section by subjecting a suitable hydrocarbon feedstock to a reforming process including a partial oxidation to yield a gaseous mixture of carbon oxides and hydrogen. Such mixture is commonly termed syngas or make-up gas.

The syngas after cooling and compression to methanol synthesis conditions is fed to a methanol synthesis loop wherein crude methanol is synthesised in a gas-solid catalytic reactor over a copper-based catalysts CuO/ZnO/Al₂O₃. Typical operating temperature and pressure of the synthesis reactor are 250 °C to 300 °C and 50 to 100 bar.

A methanol synthesis loop produces typically a stream of raw methanol and a purge gas removed from the loop. The purge gas contains unreacted hydrogen and inert compounds such as methane, argon and nitrogen. It is known to recover unreacted hydrogen from the purge gas, e.g. by means of a hydrogen recovery unit (HRU). The HRU typically operate by membrane separation or pressure swing adsorption (PSA).

The composition of the syngas feed to the methanol reactor is characterised by the following stoichiometric number:

R = (H₂ - CO₂) / (CO + CO₂).

A stoichiometric number at about 2.02 to 2.1 is generally considered optimal for most of the catalysts used in the methanol synthesis.

Based on the technology adopted, the syngas produced in the front-end may have a sub-stoichiometric content of hydrogen. This term denotes that the make-up gas contains less hydrogen than a target stoichiometric amount for methanol synthesis. In most cases for example a syngas stream having a stoichiometric number R lower than 2 is considered sub-stoichiometric.

Typically, a make-up gas with such deficiency of hydrogen is produced from stand-alone autothermal reforming fired with oxygen or oxygen-enriched air and running at a relatively low steam to carbon ratio. This technique of producing the make-up gas is interesting for its low consumption of fuel, however the drawback of the hydrogen deficit must be resolved.

A lack of hydrogen may result in a low efficiency and high generation of undesirable by-products such as higher alcohols and ketones. There is the need therefore to adjust the hydrogen content of the make-up gas before its introduction in the methanol reactor.

The hydrogen separated from the purge gas of the synthesis loop, as above described, can be used for this purpose. Unfortunately, due to the low amount of purge gas (i.e. not greater than 5%), the hydrogen that can be recovered from the loop purge may not be sufficient to reach the desired stoichiometric number in the feed.

Various attempts have been made in the prior art to solve this problem. US6797252 discloses a process for producing synthesis gas wherein the composition of the make-up gas is adjusted by means of a plurality of secondary reactors wherein at least one of the secondary reactors is a low-temperature water gas shift reactor and the other is a high-temperature water gas shift reactor. In the prior art, the provision of a low-temperature water gas shift reactor arranged in series after a high-temperature water gas shift reactor is deemed essential to recover the amount of hydrogen necessary to adjust the stochiometric number of the make-up gas feeds to the methanol reactor.

The water-gas shift reactor converts carbon monoxide and water into hydrogen and carbon dioxide. Hydrogen is separated from carbon dioxide via a CO₂ removal unit and is then fed to the methanol reactor to adjust the stoichiometric number.

This technique however requires the installation of two expensive water gas shift units; another drawback is the amount of hydrogen recovered in the WGS units is not constant during the life of the installation, due to the partial deactivation of the catalyst over time, and the stochiometric number progressively deviates from the optimum value. Currently, no methods or processes are available that allow to adjust and maintain the stoichiometric number of the syngas fed to the methanol reactor to the optimal value during the entire production cycle.

Additionally, conventional methanol synthesis processes are subject to an additional disadvantage when the autothermal reformer is operated at low steam to carbon molar ratio (S/C) and when a relatively high pressure is adopted e.g. S/C lower than 1.5 and pressure of about 35 to 50 bar, because under these operating conditions a relative a high content of unconverted methane is retained in the syngas.

Such unconverted stream is usually extracted from the methanol converter as a purge gas and after separation from hydrogen it can be recycled as fuel to fired heater. However, in case of a large amount of such waste gas to be valorised as fuel, the heating duty necessary for the process can be saturated by the waste gases alone, leading to issues of operability of the fired heater. As a result, not all of the methane-containing waste gas can be advantageously combusted. Therefore, it is desirable to find a more efficient way to exploit the methane stream. GB2585477A discloses a methanol synthesis process.

Therefore, in light of the drawbacks set out above, it is desirable to design a methanol process that can exploit in a more efficient way the unconverted methane stream meanwhile allowing the adjustment of the stoichiometric number of the syngas fed to the methanol reactor.

### Summary of the invention

The invention faces the problem of how to deal with the high content of methane retained in the purge gas stream extracted from the methanol synthesis loop. Additionally, the invention faces the problems on how to adjust the hydrogen content in the make-up gas for the synthesis of methanol, when the make-up gas is generated in a sub-stoichiometric condition. More specifically, the invention aims to provide a process wherein the synthesis loop can run efficiently when the syngas produced in the front-end has a sub-stoichiometric hydrogen content and a significant methane content.

Another aim is to provide a process wherein the adjustment of the hydrogen content in the feed gas can be controlled in a flexible way to conform to different operating condition, particularly to ageing and loss of efficiency of the HTS catalyst over time and variability of the feedstock used as a hydrocarbon source. A further aim is to develop a methanol plant with low capital expenditure (CAPEX) and low natural gas consumption. Still a further aim is to develop a process and plant which is scalable to a large capacity, e.g. of 10'000 tons/day of methanol, at economically attractive conditions. A further aim is to reduce emissions so that a large plant is also environmentally acceptable.

The above aims are reached with a process and a plant according to the claims.

In the process of the invention a portion of the make-up gas obtained by reforming is separated during a cooling process and before the cooling process is completed, so that it still contains a significant amount of water. This separated partially cooled make-up gas is subject to water-gas shift and hydrogen recovery, thus obtaining a first hydrogen stream. The remainder of the make-up gas is further cooled obtaining a fully cooled main stream of make-up gas.

A second hydrogen stream is obtained from the methanol synthesis loop purge. The first and the second hydrogen streams are added to the main stream of make-up gas to adjust its hydrogen content. The addition of the first hydrogen stream and second hydrogen stream can be performed at the same location or different locations.

Additionally, a methane-rich stream is also obtained from the loop purge, which is recycled as a feedstock to the reforming process for the production of make-up gas.

The separated portion of makeup gas is preferably a minor portion. Particularly preferably, this portion may be (in volume) not greater than 15% of the make-up gas available from the reforming process, for example 1% to 10%.

The invention provides a dual recovery of hydrogen by means of the shift and hydrogen purification performed on the separated portion of make-up gas, and the hydrogen recovery on the synthesis loop purge stream. This dual recovery allows more flexible control in the adjustment of the hydrogen content of the main stream of make-up gas.

The separated stream of fresh make-up gas represents an additional degree of freedom that can be advantageously oversized to tackle variations in the feed composition, granting good feedstock flexibility, as well as flexibility from start of run (SOR) to end of run (EOR) conditions of the HTS catalyst.

Particularly, an advantage of the invention is that said dual recovery of hydrogen is combined with recycle of a methane-rich stream obtained from the synthesis loop purge.

The shift of the separated portion of fresh make-up gas allows advantageous heat recovery, due to the elevated temperature of said stream.

The methane rich stream recovered from the loop purge may be obtained at a pressure level compatible with the syngas generation section, particularly when a membrane separation is adopted. Accordingly, the methane-rich stream can be recycled without recompression.

Preferably the first hydrogen stream is obtained with a PSA process and the second hydrogen stream is obtained with membrane separation. Obtaining the first hydrogen stream with a PSA process has further advantages. The PSA process recovers a pure hydrogen stream which is kept at the same pressure as the feed, therefore allowing mixing with the remaining part of the synthesis gas without any recompression. Additionally, the hydrogen stream produced by the PSA has a very high purity, so that a part of it can be exported for other uses, for example for ammonia co-production.

The reforming process may include autothermal reforming, optionally preceded by one or more pre-reforming steps.

The invention is particularly interesting when the makeup gas is generated by autothermal reforming. The dual hydrogen recovery and methane recovery of the invention are well suited for integration with autothermal reforming, even more when the hydrogen recovery on the synthesis loop purge is performed using a membrane based HRU, allowing the recycle without recompression of part of the methane rich retentate as feed for the autothermal reforming.

Still another aspect of the invention in the application is subjecting at least a portion of the make-up gas characterised by a low steam to dry gas ratio to a high-temperature water gas shift conversion step employing high-temperature water gas shift catalysts suitable to operate at low steam to dry gas ratio e.g. from 0.1 to 0.5 wherein the steam to dry gas ratio is defined as follow S/DG=H₂O/(1-H₂O).

### Preferred embodiments

The cooling of the makeup gas may be performed in a cooling zone comprising a plurality of heat exchangers which are arranged in series to form a cooling train so that for each pair of consecutive first and second heat exchangers of the cooling train, the effluent of a first heat exchanger is further cooled in the second heat exchanger. In such a case, a minor portion of make-up gas is separated at an intermediate point of the cooling zone before passage in at least one of the heat exchangers. Accordingly, the separated portion of makeup gas does not fully traverse the cooling train and is not completely cooled. The remainder of makeup gas is further cooled in the cooling zone to produce the fully cooled main stream of makeup gas.

For practical reasons, a preferred embodiment includes two heat exchanger sections in series wherein the minor portion of make-up gas is separated after a passage in the first heat exchanger section and prior to entering the second heat exchanger section. The first heat exchanger section is configured to partially cool down the make-up gas obtained from reforming. The second heat exchanger section is configured to completely cool down the remainder of make-up gas, after separation of the minor portion directed to shift and hydrogen separation.

The temperature of the minor portion of gas after separation is preferably 320 to 450 °C. The temperature of the second portion of gas, after complete cooling, is preferably 25 to 60 °C more preferably 45 °C.

Preferably, the cooling of the make-up gas is carried out by means of indirect heat transfer with water or steam. The heat exchanger sections may comprise one or more of a steam generator, a steam superheater and/or a water preheater.

In an embodiment, a hot water produced in the makeup gas cooling zone may be feed as a reagent to the reforming process.

As mentioned above, particularly preferred application of the invention concerns the generation of make-up gas by autothermal reforming, optionally after pre-reforming. Autothermal reforming is performed in a suitable autothermal reformer (ATR) in the presence of oxygen or oxygen-containing gas (air or enriched air), steam and optionally carbon dioxide over a suitable catalyst under oxidation conditions effective to produce the make-up gas. Pre-reforming, if provided, may be performed in one or more pre-reformers.

In a preferred embodiment, the autothermal reformer operates at low steam to carbon ratio (S/C) preferably comprised between 0.5 and 1.5, more preferably between 0.8 and 1.2. Preferably, the autothermal reformer operates at a pressure between 25 and 60 abs bar, and more preferably between 35 and 50 abs bar.

The water-gas shift of the separated portion of make-up gas may include catalytic high-temperature shift (HTS). A high-temperature shift may be performed at a temperature in the range 300 to 500 °C, preferably 350 to 450 °C. Preferably the water-gas shift of the separated portion is performed solely in a high-temperature shift reactor without a subsequent medium-temperature or low-temperature shift.

The first hydrogen recovering section includes preferably a Pressure Swing Adsorption (PSA) unit. The second hydrogen recovery section includes preferably a membrane-based hydrogen purification unit.

In an interesting embodiment, part of the hydrogen recovered from the PSA may be used as a fuel to meet the energy demand of the process, and/or as feedstock for the coproduction of ammonia and/or employed for other processes outside the synthesis of ammonia. The use of hydrogen from PSA as a fuel may further reduce the carbon emission of the process.

More specifically, the use of hydrogen recovered from the PSA ("PSA hydrogen") as a fuel gives the following advantages: the purified hydrogen obtained from the PSA can fuel the fired heaters of the plant, instead of the light hydrocarbons that are usually employed both as feedstock and fuel. The direct emissions of the plant can be reduced significantly, or eliminated completely by foreseeing a carbon capture step. The carbon to be captured remains in concentrated form in process streams at high pressure, instead of being diluted in low-pressure flue gases, making the carbon capture step less expensive.

The use of PSA hydrogen for ammonia co-production is also possible thanks to the high purity of hydrogen that can be produced by PSA. The highly pure PSA hydrogen stream is well suited for direct use in an ammonia synthesis loop where the specification on the hydrogen purity is very strict due to poisoning issues of the ammonia synthesis catalyst.

The ability to recover hydrogen from two independent sources, namely a portion of make-up gas and the loop purge, allows high flexibility in the selection of the hydrocarbon source fed to the autothermal reactor. For example in the event that the amount of H₂ recovered from the separated portion of make-up gas is reduced due to the deposition of carbonaceous product such as coke or soot over the catalytic surface in the WGS reactor, this reduction can be compensated by increasing the amount of H₂ recovered from loop purge.

Likewise, in the event that the feedstock should be changed to a hydrocarbon source characterised by a higher carbon-to-hydrogen ratio, the consequent reduction of the stoichiometric number can be compensated by increasing the amount of make-up gas fed to the HTS and related hydrogen recovery unit.

The hydrocarbon source is preferentially a light hydrocarbon source and more preferentially a natural gas. In an embodiment of the present invention, the hydrocarbon source may be subjected to hydrodesulfurisation, and subsequently to a first and to a second pre-reforming stage. Preferentially, the first pre-reforming is carried out in an adiabatic fixed-bed reactor at a temperature of approximately 350 to 530 °C; the secondary pre-reforming may be carried out in an adiabatic reactor at a temperature higher than that of the first pre-reforming reactor. Preferentially, the temperature of the second pre-reformer is approximately 500 to 750 °C.

Advantageously, treating the hydrocarbon source in a first pre-reformer and in a second pre-reformer that operates at a higher temperature over the first one, enables a high conversion of the high molecular weight hydrocarbons (>C₂) to methane in the pre-reformer and conversion of methane to syngas in the second pre-reformer thus deposition of soot over the catalyst surface in the autothermal reformer is avoided and the life span of the reforming catalytic is extended. Moreover, the oxygen requirement for the operation of the autothermal reformer is minimised due to the additional reforming duty adsorbed by the secondary pre-reformer.

In another embodiment, the hydrocarbon source is fed to the desulphurisation unit and subsequentially to a single pre-reformer before being feed to the autothermal reformer.

In another embodiment, the hydrocarbon source is fed to the desulphurisation unit and subsequentially to the autothermal reformer and no pre-reforming stage is employed.

The autothermal reforming may be fired with oxygen or enriched air produced in an air separation unit. A further aspect of the invention is the integration of such air separation unit with the other equipment. The high-pressure steam produced from cooling of the makeup gas may be used to operate the air separation unit, e.g. to power a steam turbine of the unit. A medium-pressure steam extracted from the air separation unit may be further used by injection upstream the pre-reformer, therefore serving as the process steam necessary in the reforming section. Advantageously, the totality of the steam produced in the process is exploited in the plant, therefore an improved design flexibility is envisaged in concomitant with an improved efficiency due to a reduction in the natural gas consumptions as a result of the employment of an autothermal reformer that operates at low steam to carbon S/C ratio.

Furthermore, the steam demand and production between the syngas generation and the methanol synthesis are decoupled to improve the operability and the flexibility of the plant.

A particularly preferred embodiment includes:
a light hydrocarbon feed is converted to syngas in the following steps: hydrodesulphurization, pre-reforming, secondary pre-reforming (at higher temperature), autothermal reforming;
the autothermal reforming is performed at low S/C ratios 0.5-1.5 and at a pressure of 25-60 bar, the inlet stream to the autothermal reformer consists of
the pre-reformed feed mixed with a portion of the HRU retentate;
the resulting syngas is cooled by raising high pressure steam;
a portion of the resulting fresh make-up gas is withdrawn and sent to a water-gas shift reactor, the water-gas shift effluent is then subjected to H₂ recovery by PSA;
the resulting stream of pure hydrogen, obtained from the PSA, is mixed with the remaining larger part of the fresh make-up gas and with a hydrogen enriched stream from a membrane HRU unit, to form an adjusted make-up gas that is fed to the synthesis loop compressor;
the compressed adjusted make-up gas is mixed with the recirculated recycle stream and fed to a methanol reactor;
the reactor effluent is cooled and a crude methanol is condensed and sent to a distillation section;
a purge stream is withdrawn from the unreacted gas mixture;
the remainder (and majority) of the unreacted gas mixture (recycle stream) is recycled through a recirculatory to the inlet of the methanol reactor, by mixing with the adjusted make-up gas;
the purge stream is sent to a membrane-based HRU, the retentate depleted of hydrogen is partly discarded and used as fuel, and partly recycled to the autothermal reformer;
the hydrogen enriched permeate is mixed with the fresh make-up gas and the pure H₂ from PSA to form the adjusted make-up gas.

According to still another aspect of the invention, the makeup gas has a low steam to dry gas (SlOG) ratio. For example this ratio in in the range 0.1 to 0.5. In case of a makeup gas with such low S/DG ratio the shift reaction is preferably performed over an iron-free catalyst.

The preferred embodiments of the invention give among others the following advantages: consumption is low due to the low S/C used in the front-end; the requirement of oxygen for the operation of the autothermal reformer can be minimized thanks to the additional reforming duty absorbed by the secondary pre-reforming; the production of steam can be limited due to the low S/C used in the front-end so that no steam must be exported.

The steam network may be arranged in order to take advantage of the low steam production. In particular, steam demand and production between the syngas generation and the methanol synthesis sections of the plant can be decoupled, thus increasing the plant operability and modularity.

Preferably the crude methanol extracted from the methanol synthesis loop is refined to high purity methanol by a distillation scheme comprising four columns. Preferably, the four columns comprise a topping column for the removal of volatile components present in the crude, and three refining columns separating methanol from water and higher alcohol by-products. Advantageously, the four columns distillation layout yields refined methanol by using a smaller amount of steam for reboiling. The decreased steam is necessary to capitalize on the reduced gas consumption and steam production provided by the low S/C front end.

Preferably, the pressure levels of the three columns are respectively in the ranges of 12 to 16 bar, 6 to 10 bar and 0.5 to 3 bar. Advantageously, the related heat necessary for reboiling can be provided at a temperature level compatible with low-pressure steam obtained from the operation of the steam turbines.

Even more advantageously, the amount of reboiling duty for the distillation recovered from the make-up gas cooling may be minimised and limited to the topping reboiling duty. Refining duty may be obtained from the condensation of steam extracted from the operation of a steam turbine. Consequently, the steam demand and production between the syngas generation and the methanol synthesis sections of the plant may be decoupled and the plant operability and modularity may be increased.

In an embodiment, the high-pressure steam produced in the syngas cooling section/zone is utilised to operate an air separation unit that provides pure oxygen fed to the autothermal reformer. Medium pressure steam may be extracted from the steam turbine of the air separation unit and utilised in part as process steam to carry out the reforming reactions of the syngas generation section, and in another part to operate further steam turbines for plant services.

In an embodiment of the invention, the steam generated by heat exchange in the methanol synthesis reactor is superheated in a fired heater and utilised to operate the adjusted make-up gas compressor and the recycle stream recirculatory. Preferably, low-pressure steam is extracted from the make-up gas compressor steam turbine and condensed in the primary reboiler of the first refining column.

### Description of the figures

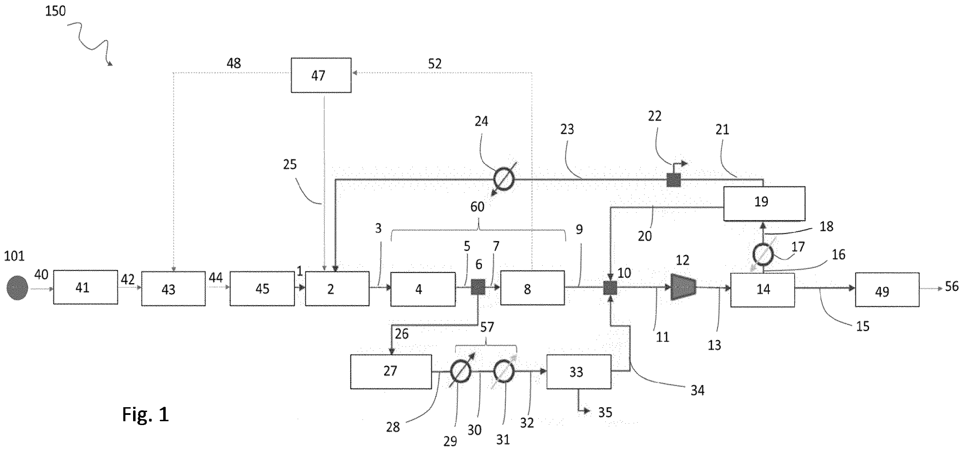

Fig. 1 shows a methanol synthesis process according to an embodiment of the invention.

### Detailed description of the preferred embodiments

Fig. 1 illustrates a scheme 150 of a plant for producing methanol 56 from a light hydrocarbon 101, e.g. natural gas.

The hydrocarbon 101 is supplied via line 40 to a hydrodesulfurisation unit 41 and the sulfur-free hydrocarbon 42 is fed to a first pre-reformer 43 wherein high molecular weight hydrocarbons (≥C₂) are partially converted into methane, hydrogen and carbon oxides.

The gas mixture 44 leaving the pre-reforming is then fed to a second pre-reformer 45 wherein further conversion of the hydrocarbons is carried out yielding a hydrocarbon-containing gas 1 predominantly comprising methane, hydrogen and carbon oxides.

The hydrocarbon containing gas 1 is fed with an oxygen-containing gas 25 to an autothermal reformer 2 to yield a make-up gas 3 having a sub-stoichiometric contend of hydrogen, that is a hydrogen deficit over a stoichiometric number prescribed by the methanol reaction.

The oxygen-containing gas 25 is obtained from an air separation unit 47. The air separation unit 47 is operated with high-pressure stream 52 obtained in a makeup gas cooling zone 60. A medium-pressure steam 48 discharged by the air separation unit 47 is introduced in the first pre-reformer 43 and used as process steam further down the line (not shown).

The hot make-up gas 3 exiting the reformer 2 is fed to a cooling zone 60 which, in the shown embodiment, includes a first heat exchanger section 4 and a second heat exchanger section 8.

In the first heat exchanger section 4, the hot make-up gas 3 is cooled to yield a partially cooled make-up gas 5 and the high-pressure stream 52 is produced.

A minor fraction 26 of the partially cooled make-up gas 5 is separated from an intermediate point 6 of the cooling zone 60, namely after passage in the first heat exchanger section 4 and before entering the second heat exchanger section 8. Said separated portion 26 is sent to a train including a HTS shift reactor 27, a cooling section 57 and a PSA unit 33 for separation of a hydrogen stream 34. The separated stream 26 is preferably about 2% of the makeup gas 5.

The remaining portion 7 of makeup gas (after separation of the above fraction 26) is sent to the second heat exchanger section 8 where it is further cooled obtaining a stream 9 of fully cooled makeup gas.

More in detail the separated makeup gas 26 is fed to the high-temperature shift reactor 27 to yield a shifted gas 28 enriched in hydrogen. Said shifted gas 28 is then cooled in the cooling section 57 comprising a first heat exchanger 29 and a second heat exchanger 31. The effluent 30 of the first heat exchanger 29 is further cooled in the second heat exchanger 31. The so obtained cooled gas 32 is fed to the pressure swing adsorption unit 33 to yield the hydrogen stream 34 and a tail gas 35 comprising methane and carbon dioxides. Said tail gas 35 may be sent to combustion.

The temperature of the make-up gas 3 leaving the autothermal reformer 2 may be about 1000 °C. The temperature of the separated make-up gas 26 at the inlet of the high-temperature water gas shift reactor 27 is typically about 350 °C. The temperature of the shifted gas 28 leaving the water gas shift reactor 27 may be about 470 °C and the temperature of the cooled gas 32 entering the pressure swing adsorption unit 33 after proper cooling is of about 45 °C.

The fully cooled make-up gas 9 is mixed at a mixing point 10 with at least a portion of the hydrogen stream 34 and with a permeate 20 rich in hydrogen which is recovered from the loop purge as described below. By mixing with the hydrogen stream 34 and permeate 20, the content of hydrogen in the makeup gas 9 is adjusted, i.e. the initial lack of hydrogen is compensated.

Typically, a hydrogen stream obtainable in a PSA unit has a high purity. It should be noted the hydrogen stream 34 may contain unavoidable impurities.

The so obtained adjusted make-up gas 11 after compression in a syngas compressor 12 is fed to a methanol synthesis loop 14.

In some embodiments the hydrogen stream 34 leaving the pressure swing adsorption unit 33 may be fed directly to the mixing point 10 of the main line, i.e. no compression stage is required as the hydrogen stream 34 is extracted at a sufficient pressure.

A condensate crude methanol stream 15 and a purge stream 16 are extracted from the methanol synthesis loop 14. The condensate crude methanol stream 15 is purified in a distillation section 49 to yield a pure methanol stream 56.

Preferably the distillation section 49 includes four distillation columns operating in cascade. Particularly preferably the four-column setup described in EP 2 617 478 may be adopted, which has the advantage of a low consumption of steam.

The purge stream 16 after suitable cooling in a heat exchanger 17 is fed to a membrane-based hydrogen purification system 19 obtaining a permeate 20 rich in hydrogen and a retentate 21 rich in methane. Typically, the H₂ recovery in the hydrogen purification system 19 is carried out so that the recovered H₂ stream 20 is at a pressure compatible with direct mixing with stream 9.The permeate 20 rich in hydrogen is recirculated back to the mixing point 10 where it concurs to adjustment of the hydrogen content in the makeup gas 9.

At least a portion of the retentate 21 may be fed, after cooling in a heat exchanger 24, to the reformer 2 and used as an additional feedstock for the synthesis of the make-up gas 3. The recycle of the retentate 21 rich in methane tackles the problem of the methane slip and avoid the saturation of the heating duty by the waste gases of the plant. A portion 22 of the retentate may be separated and sent to combustion.

## Claims

1. A process for producing methanol comprising the following steps:
a. reforming of a hydrocarbon-containing source into a make-up gas (3) comprising hydrogen, carbon oxides and water;
b. subjecting the make-up gas (3) generated in step (a) to a cooling process;
c. separating a portion (26) of makeup gas during the cooling process of step b) and before the cooling process is completed, and subjecting the remainder portion (7) of makeup gas to complete cooling, obtaining a main stream of makeup gas (9), the separated gas (26) being at a higher temperature than the main stream (9);
d. subjecting said separated makeup gas (26) to at least one water gas shift (WGS) conversion step (27) obtaining a shifted gas (28) enriched in hydrogen;
e. cooling said shifted gas (28) and feeding the cooled shifted gas (32) to a first hydrogen recovery section (33) obtaining a first hydrogen stream (34);
f. adding the main stream of makeup gas (9) with said first hydrogen stream (34) and with a second hydrogen stream (20) obtained in step (i), thus obtaining an adjusted make-up gas (11) with an adjusted content of hydrogen;
g. feeding the adjusted make-up gas (11) to a methanol synthesis loop (14) wherein catalytic conversion of carbon oxides to methanol is carried out under methanol synthesis conditions, obtaining a condensate crude methanol stream (15);
h. purifying the condensate crude methanol stream (15), preferably in a distillation section (49), obtaining a methanol product (56);
i. feeding a purge stream (16) withdrawn from the methanol synthesis loop to a second hydrogen recovery section (19) obtaining a second hydrogen stream (20) containing hydrogen removed from the purge stream, and a tail gas (21) containing methane;
j. adding the second hydrogen stream (20) to the main stream of makeup gas (9) according to step (f);
k. using at least a portion of the tail gas (21) as a feedstock for the production of the make-up gas (3) of step (a).

2. A process according to claim 1 wherein said separated portion (26) of makeup gas is a minor portion.

3. A process according to claim 2 wherein the volumetric flow rate of said separated portion of makeup gas (26) is not greater than 15% of the total volumetric flow rate of said make-up gas (3), preferably 1% to 10%.

4. A process according to any of claims 1 to 3 wherein the cooling process of step c) is performed in a cooling section (60) comprising a plurality of heat exchangers which are arranged in series and said separated portion (26) of makeup gas is separated after the passage in at least one of the heat exchangers.

5. A process according to any previous claims, wherein the reforming of step a) includes autothermal reforming, optionally preceded by pre-reforming.

6. A process according to claim 5, wherein the autothermal reforming is performed at a steam to carbon ratio (S/C) comprised between 0.5 and 1.5, preferably between 0.8 and 1.2.

7. A process according to claim 5 or 6 wherein the autothermal reforming is performed at an absolute pressure between 25 and 60 bar, preferably between 35 and 50 bar.

8. A process according to any of the previous claims, wherein the water-gas shift conversion of step d) includes high-temperature shift, preferably between 300 and 500 °C, more preferably between 350 and 450 °C.

9. A process according to any of the previous claims, wherein the first hydrogen recovery section (27) includes a pressure swing adsorption unit.

10. A process according to any of the previous claims, wherein the second hydrogen recovery section (19) includes a membrane-based hydrogen recovery unit.

11. A process according to any of the previous claims, wherein a portion of said first hydrogen stream (34) is used as a fuel to meet the energy demand of the process and/or as feedstock for the coproduction of ammonia.

12. A process according to any of the previous claims, wherein said hydrocarbon containing-gas is obtained from a natural gas source (101) by hydrodesulfurisation (41), pre-reforming (43) and secondary pre-reforming (45) wherein said secondary pre-reforming (45) is carried out a temperature higher than said pre-reforming (43).

13. A process according to any of the previous claims, wherein the reforming of step a) is performed with oxygen or an oxygen-containing stream produced in an air separation unit (47) and a steam (52) generated in the makeup gas cooling of step b) is used to operate said air separation unit.

14. A process according to any of the previous claims wherein the makeup gas (3) obtained in the reforming process has a steam to dry gas ratio not greater than 0.5, preferably 0.1 to 0.5.

15. A process according to any previous claims wherein the purification of the condensate crude methanol stream (15) of step (h), is carried out in a distillation section (49) comprising four columns operating in cascade, wherein one of the four columns is a topping column for the removal of volatile components, and the other three columns are refining columns designed to separate methanol from water and higher alcohol by-products.

16. A plant for producing methanol from a synthesis gas containing hydrogen, carbon oxides and optionally inert components, comprising:
a) a reforming section suitable for reforming a hydrocarbon-containing source into a make-up gas (3) comprising hydrogen, carbon oxides and water;
b) a cooling section arranged to cool the make-up gas (3) generated in step (a);
c) a line arranged to separate a portion (26) of makeup gas from an intermediate location of said cooling section and before complete cooling and a line arranged to subject the remainder portion (7) of makeup gas to complete cooling in the section, obtaining a main stream of fully cooled makeup gas (9) at a temperature lower than the separated makeup gas (26);
d) a water gas shift section (27) connected to said line carrying the separate portion of makeup gas (26) and configured to produce a shifted gas (28) enriched in hydrogen;
e) a cooling section of the shifted gas and a first hydrogen recovery section (33) arranged to receive said shifted gas after cooling and to produce a first hydrogen stream (34);
f) a line arranged to add said first hydrogen stream (34) to the main stream of makeup gas (9) and a line arranged to add a second hydrogen stream (20) obtained in step (i) to said makeup gas, thus obtaining an adjusted make-up gas (11) with an adjusted content of hydrogen;
g) a methanol synthesis loop (14) and a line arranged to feed the adjusted make-up gas (11) to said loop wherein catalytic conversion of carbon oxides to methanol is carried out under methanol synthesis conditions, obtaining a condensate crude methanol (15);
h) a purification section of the condensate crude methanol (15), preferably a multi-column distillation section (49), obtaining methanol (56);
i) a second hydrogen recovery section (19) arranged to receive a purge stream (16) withdrawn from the methanol synthesis loop and to obtain said second hydrogen stream (20) and a tail gas (21) containing methane removed from the purge stream;
j) a line arranged to feed at least a portion of the tail gas (21) as a feedstock to the reforming section for the production of the make-up gas (3).

17. A plant according to claim 16, including one or more of the following:
the reforming section includes an autothermal reformer, optionally with one or more pre-reformer(s);
the first hydrogen recovery section is a PSA unit;
the second hydrogen recovery section is a membrane separation unit;
said water-gas shift section of the separated makeup gas includes a high-temperature shift reactor.

18. A plant according to claim 16 or 17, wherein the purification section of the condensate crude methanol (15) of step (h) comprises four columns operating in cascade, wherein one of the four columns is a topping column for the removal of volatile components, and the other three columns are refining columns designed to separate methanol from water and higher alcohol by-products.

## Patentansprüche

1. Verfahren zur Methanolherstellung, umfassend die folgenden Schritte:
a. Reformieren einer kohlenwasserstoffhaltigen Quelle zu einem Zusatzgas (3), umfassend Wasserstoff, Kohlendioxide und Wasser;
b. Unterziehen des in Schritt (a) erzeugten Zusatzgases (3) einem Kühlprozess;
c. Abtrennen eines Teils (26) des Zusatzgases während des Kühlvorgangs von Schritt b) und vor Beendigung des Kühlvorgangs, und Unterziehen einer vollständigen Kühlung des restlichen Teils (7) des Zusatzgases, um einen Hauptstroms von Zusatzgas (9) zu erhalten, wobei das abgetrennte Gas (26) eine höhere Temperatur hat als der Hauptstrom (9);
d. Unterziehen des abgetrennten Zusatzgases (26) mindestens einem Wassergas-Shift (WGS) Umwandlungsschritt (27), um ein mit Wasserstoff angereichertes Shift-Gas (28) zu erhalten;
e. Kühlen des Shift-Gases (28) und Zuführen des gekühlten Shift-Gases (32) zu einem ersten Wasserstoffrückgewinnungsabschnitt (33), der einen ersten Wasserstoffstrom (34) erhält;
f. Hinzufügen eines ersten Wasserstoffstrom (34) und eines in Schritt (i) erhaltenen zweiten Wasserstoffstroms (20) zu dem Hauptstroms des Zusatzgases (9), um ein eingestelltes Zusatzgas (11) mit einem eingestellten Gehalt an Wasserstoff zu erhalten;
g. Einspeisen des eingestellten Zusatzgases (11) in einen Methanolsynthesekreislauf (14), wobei die katalytische Umwandlung von Kohlenstoffoxiden zu Methanol unter Methanolsynthesebedingungen durchgeführt wird, um ein Kondensat-Rohmethanolstrom (15) zu erhalten;
h. Reinigen des kondensierten Rohmethanolstroms (15), vorzugsweise in einem Destillationsabschnitt (49), um ein Methanolprodukt (56) zu erhalten;
i. Zuführen eines aus dem Methanolsynthesekreislauf abgezogenen Spülstroms (16) zu einem zweiten Wasserstoffrückgewinnungsabschnitt (19), um einen zweiten Wasserstoffstrom (20) zu erhalten, der aus dem Spülstrom entfernten Wasserstoff enthält, und ein Methan enthaltendes Abgas (21);
j. Zugabe des zweiten Wasserstoffstroms (20) zum Hauptstrom des Zusatzgases (9) gemäß Schritt (f);
k. Verwendung mindestens eines Teils des Abgases (21) als Ausgangsmaterial für die Herstellung des Zusatzgases (3) von Schritt (a).

2. Verfahren nach Anspruch 1, wobei der abgetrennte Teil (26) des Zusatzgases ein kleiner Teil ist.

3. Verfahren nach Anspruch 2, bei dem der Volumendurchsatz des abgetrennten Teils des Zusatzgases (26) nicht mehr als 15 % des Gesamtvolumendurchsatzes des Zusatzgases (3) beträgt, vorzugsweise 1 % bis 10 %.

4. Verfahren nach einem der Ansprüche 1 bis 3, wobei der Kühlprozess von Schritt c) in einem Kühlabschnitt (60) durchgeführt wird, der eine Mehrzahl von Wärmetauschern umfasst, die in Reihe angeordnet sind, und der abgetrennte Teil (26) des Zusatzgas nach dem Durchgang durch mindestens einen der Wärmetauscher abgetrennt wird.

5. Verfahren nach einem der vorhergehenden Ansprüche, wobei das Reformieren von Schritt a) ein autothermes Reformieren umfasst, dem optional ein Vorreformieren vorausgeht.

6. Verfahren nach Anspruch 5, wobei die autotherme Reformierung bei einem Dampf-Kohlenstoff-Verhältnis (S/C) zwischen 0,5 und 1,5, vorzugsweise zwischen 0,8 und 1,2, durchgeführt wird.

7. Verfahren nach Anspruch 5 oder 6, wobei die autotherme Reformierung bei einem Absolutdruck zwischen 25 und 60 bar, vorzugsweise zwischen 35 und 50 bar, durchgeführt wird.

8. Verfahren nach einem der vorhergehenden Ansprüche, wobei die Wassergas-Shift-Umwandlung von Schritt d) eine Hochtemperaturverschiebung umfasst, vorzugsweise zwischen 300 und 500 °C, besonders bevorzugt zwischen 350 und 450 °C.

9. Verfahren nach einem der vorhergehenden Ansprüche, wobei der erste Wasserstoffrückgewinnungsabschnitt (27) eine Druckwechseladsorptionseinheit umfasst.

10. Verfahren nach einem der vorhergehenden Ansprüche, wobei der zweite Wasserstoffrückgewinnungsabschnitt (19) eine membranbasierte Wasserstoff-Rückgewinnungseinheit umfasst.

11. Verfahren nach einem der vorhergehenden Ansprüche, wobei ein Teil des ersten Wasserstoffstroms (34) als Brennstoff verwendet wird, um den Energiebedarf des Verfahrens und/oder als Ausgangsmaterial für die Koproduktion von Ammoniak zu verwenden.

12. Verfahren nach einem der vorhergehenden Ansprüche, wobei das kohlenwasserstoffhaltige Gas aus einer Erdgasquelle (101) erhalten wird durch Hydrodesulfurierung (41), Vorreformierung (43) und sekundäre Vorreformierung (45), wobei die sekundäre Vorreformierung (45) bei einer Temperatur durchgeführt wird, die höher ist als die Vorreformierung (43).

13. Verfahren nach einem der vorhergehenden Ansprüche, wobei die Reformierung von Schritt a) mit Sauerstoff oder einem sauerstoffhaltigen Strom durchgeführt wird, der in einer Luftzerlegungseinheit (47) erzeugt wird, und ein in der Zusatzgas Kühlung von Schritt b) erstellter Dampf (52) zum Betreiben der Luftzerlegungseinheit verwendet wird.

14. Verfahren nach einem der vorhergehenden Ansprüche, bei dem das bei der Reformierung erhaltene Zusatzgas (3), ein Verhältnis von Dampf zu Trockengas von nicht mehr als 0,5 aufweist, vorzugsweise 0,1 bis 0,5.

15. Verfahren nach einem der vorhergehenden Ansprüche, wobei die Reinigung des Kondensat-Rohmethanolstroms (15) aus Schritt (h) in einem Destillationsabschnitt (49) durchgeführt wird, der vier in Kaskade arbeitende Kolonnen umfasst, wobei eine der vier Kolonnen eine Vor-Kolonne zur Entfernung von flüchtigen Bestandteilen ist und die anderen drei Kolonnen Raffinationskolonnen zur Abtrennung des Methanols von Wasser und höheren Alkoholnebenprodukten sind.

16. Anlage zur Herstellung von Methanol aus einem Synthesegas, das Wasserstoff, Kohlenstoffoxide und optional inerte Komponenten enthält, umfassend:
a) einen Reformierungsabschnitt, der für die Reformierung einer kohlenwasserstoffhaltigen Quelle in ein Zusatzgas (3) geeignet ist, das Wasserstoff, Kohlenstoffoxide und Wasser umfasst;
b) einen Kühlabschnitt, der zum Kühlen des in Schritt (a) erzeugten Zusatzgases (3) eingerichtet ist;
c) eine Leitung eingerichtet zum Abtrennen eines Teils (26) des Zusatzgases von einer Zwischenstelle des Kühlabschnitts und vor der vollständigen Kühlung und eine Leitung eingerichtet, um den verbleibenden Teil (7) des Zusatzgases einer vollständigen Abkühlung in dem Abschnitt zu unterziehen, wobei ein Hauptstrom von vollständig abgekühltem Zusatzgas (9) mit einer niedrigeren Temperatur als das abgetrennte Ergänzungsgas (26) erhalten wird;
d) einen Wassergas-Shift-Abschnitt (27), der mit der Leitung verbunden ist, die den separaten Teil des Zusatzgases (26) führt und so konfiguriert ist, dass er ein verschobenes Gas (28) erzeugt, das mit Wasserstoff angereichert ist;
e) einen Abschnitt zur Kühlung des Shift-Gases und einen ersten Abschnitt zur Wasserstoffrückgewinnung (33), der so angeordnet ist, dass er das Shift-Gas nach der Kühlung aufnimmt und um einen ersten Wasserstoffstrom (34) zu erzeugen;
f) eine Leitung, eingerichtet, um den ersten Wasserstoffstrom (34) zu dem Hauptstrom des Zusatzgases (9) hinzuzufügen, und eine Leitung, eingerichtet, um einen zweiten Wasserstoffstrom (20), der in Schritt (i) erhalten wurde, zu dem Zusatzgas hinzuzufügen, um ein eingestelltes Zusatzgas (11) mit einem eingestellten Gehalt an Wasserstoff zu erhalten;
g) einen Methanolsynthesekreislauf (14) und eine Leitung, eingerichtet, um das eingestellte Zusatzgas (11) dem Kreislauf zuzuführen, in dem die katalytische Umwandlung von Kohlenstoffoxiden zu Methanol unter Methanolsynthesebedingungen durchgeführt wird, wobei ein Rohmethanolkondensat (15) erhalten wird;
h) einen Abschnitt zur Reinigung des kondensierten Rohmethanols (15), vorzugsweise eine Mehrsäulendestillation (49) zur Gewinnung von Methanol (56);
i) einen zweiten Wasserstoffrückgewinnungsabschnitt (19), eingerichtet, um einen Spülstrom (16) zu empfangen, der aus dem Methanolsynthesekreislauf abgezogen wird, und um den zweiten Wasserstoffstrom (20) und ein Methan enthaltendes Abgas (21) zu erhalten, das aus dem Spülstrom entfernt wird;
j) eine Leitung, eingerichtet, um zumindest einen Teil des Abgases (21) als Ausgangsmaterial dem Reformierungsabschnitt zur Erzeugung des Zusatzgases (3) zuzuführen.

17. Anlage nach Anspruch 16, die eine oder mehrere der folgenden Eigenschaften aufweist:
der Reformierungsabschnitt umfasst einen autothermen Reformer,
oprional mit einem oder mehreren Vorreformer(n);
der erste Wasserstoffrückgewinnungsabschnitt ist eine PSA-Einheit;
der zweite Wasserstoffrückgewinnungsabschnitt ist eine Membrantrenneinheit;
der Wassergas-Shift-Abschnitt des abgetrennten Zusatzgases umfasst einen Hochtemperatur-Shift-Reaktor.

18. Anlage nach Anspruch 16 oder 17, wobei der Reinigungsabschnitt des Kondensat-Rohmethanols (15) von Schritt (h) vier in Kaskade arbeitende Kolonnen umfasst, wobei eine der vier Kolonnen eine Vor-Kolonne zur Entfernung von flüchtigen Bestandteilen ist und die anderen drei Kolonnen Raffinationskolonnen zur Abtrennung des Methanols von Wasser und höheren Alkoholnebenprodukten sind.

## Revendications

1. Procédé de production de méthanol comprenant les étapes suivantes consistant à :
a. effectuer un reformage d'une source contenant des hydrocarbures en un gaz d'appoint (3) comprenant de l'hydrogène, des oxydes de carbone et de l'eau ;
b. soumettre le gaz d'appoint (3) généré à l'étape (a) à un procédé de refroidissement ;
c. séparer une partie (26) du gaz d'appoint pendant le procédé de refroidissement de l'étape b) et avant que le procédé de refroidissement ne soit terminé, et soumettre la partie restante (7) du gaz d'appoint à un refroidissement complet, en obtenant un flux principal de gaz d'appoint (9), le gaz séparé (26) étant à une température plus élevée que celle du flux principal (9) ;
d. soumettre ledit gaz d'appoint séparé (26) à au moins une étape de conversion (27) de décalage eau-gaz (WGS) en obtenant un gaz décalé (28) enrichi en hydrogène ;
e. refroidir ledit gaz décalé (28) et introduire le gaz décalé refroidi (32) dans une première section de récupération d'hydrogène (33) en obtenant un premier flux d'hydrogène (34) ;
f. ajouter le flux principal de gaz d'appoint (9) avec ledit premier flux d'hydrogène (34) et avec un second flux d'hydrogène (20) obtenu à l'étape (i), en obtenant ainsi un gaz d'appoint ajusté (11) ayant une teneur ajustée en hydrogène ;
g. introduire le gaz d'appoint ajusté (11) dans une boucle de synthèse de méthanol (14) dans laquelle une conversion catalytique d'oxydes de carbone en méthanol est réalisée dans des conditions de synthèse de méthanol, en obtenant un flux de méthanol brut condensé (15) ;
h. purifier le flux de méthanol brut condensé (15), de préférence dans une section de distillation (49), en obtenant un produit de méthanol (56) ;
i. introduire un flux de purge (16) extrait de la boucle de synthèse de méthanol dans une seconde section de récupération d'hydrogène (19) en obtenant un second flux d'hydrogène (20) contenant de l'hydrogène retiré du flux de purge, et un gaz résiduaire (21) contenant du méthane ;
j. ajouter le second flux d'hydrogène (20) au flux principal de gaz d'appoint (9) selon l'étape (f) ;
k. utiliser au moins une partie du gaz résiduaire (21) comme matière première pour la production du gaz d'appoint (3) de l'étape (a).

2. Procédé selon la revendication 1, dans lequel ladite partie séparée (26) du gaz d'appoint est une partie mineure.

3. Procédé selon la revendication 2 dans lequel le débit volumétrique de ladite partie séparée du gaz d'appoint (26) ne dépasse pas 15 % du débit volumétrique total dudit gaz d'appoint (3), de préférence est de 1 % à 10 %.

4. Procédé selon l'une quelconque des revendications 1 à 3, dans lequel le procédé de refroidissement de l'étape c) est effectué dans une section de refroidissement (60) comprenant une pluralité d'échangeurs de chaleur qui sont agencés en série et ladite partie séparée (26) du gaz d'appoint est séparée après le passage dans au moins un des échangeurs de chaleur.

5. Procédé selon l'une quelconque des revendications précédentes, dans lequel le reformage de l'étape a) comprend un reformage autothermique, facultativement précédé d'un pré-reformage.

6. Procédé selon la revendication 5, dans lequel le reformage autothermique est effectué à un rapport vapeur sur carbone (S/C) compris entre 0,5 et 1,5, de préférence entre 0,8 et 1,2.

7. Procédé selon la revendication 5 ou 6 dans lequel le reformage autothermique est effectué à une pression absolue comprise entre 25 et 60 bar, de préférence entre 35 et 50 bar.

8. Procédé selon l'une quelconque des revendications précédentes, dans lequel l'étape de conversion d) de décalage eau-gaz comporte un décalage à haute température, de préférence entre 300 et 500 °C, plus préférablement entre 350 et 450 °C.

9. Procédé selon l'une quelconque des revendications précédentes, dans lequel la première section de récupération d'hydrogène (27) comporte une unité d'adsorption modulée en pression.

10. Procédé selon l'une quelconque des revendications précédentes, dans lequel la seconde section de récupération d'hydrogène (19) comporte une unité de récupération d'hydrogène basée sur une membrane.

11. Procédé selon l'une quelconque des revendications précédentes, dans lequel une partie dudit premier flux d'hydrogène (34) est utilisée comme combustible pour répondre à la demande énergétique du procédé et/ou comme matière première pour la coproduction d'ammoniac.

12. Procédé selon l'une quelconque des revendications précédentes, dans lequel ledit gaz contenant des hydrocarbures est obtenu à partir d'une source de gaz naturel (101) par hydrodésulfuration (41), pré-reformage (43) et pré-reformage secondaire (45), dans lequel ledit pré-reformage secondaire (45) est réalisé à une température supérieure à celle dudit pré-reformage (43).

13. Procédé selon l'une quelconque des revendications précédentes, dans lequel le reformage de l'étape a) est effectué avec de l'oxygène ou un flux contenant de l'oxygène produit dans une unité de séparation d'air (47) et une vapeur (52) générée lors du refroidissement de gaz d'appoint de l'étape b) est utilisée pour faire fonctionner ladite unité de séparation d'air.

14. Procédé selon l'une quelconque des revendications précédentes, dans lequel le gaz d'appoint (3) obtenu lors du procédé de reformage a un rapport vapeur sur gaz sec non supérieur à 0,5, de préférence de 0,1 à 0,5.

15. Procédé selon l'une quelconque des revendications précédentes dans lequel la purification du flux de méthanol brut condensé (15) de l'étape (h), est réalisée dans une section de distillation (49) comprenant quatre colonnes fonctionnant en cascade, dans lequel l'une des quatre colonnes est une colonne d'étêtage pour l'élimination de composants volatils, et les trois autres colonnes sont des colonnes de raffinage conçues pour séparer le méthanol de l'eau et des sous-produits d'alcool supérieur.

16. Usine de production de méthanol à partir d'un gaz de synthèse contenant de l'hydrogène, des oxydes de carbone et facultativement des composants inertes, comprenant :
a) une section de reformage adaptée au reformage d'une source contenant des hydrocarbures en un gaz d'appoint (3) comprenant de l'hydrogène, des oxydes de carbone et de l'eau ;
b) une section de refroidissement agencée pour refroidir le gaz d'appoint (3) généré à l'étape (a) ;
c) une conduite agencée pour séparer une partie (26) de gaz d'appoint à partir d'un emplacement intermédiaire de ladite section de refroidissement et avant refroidissement complet et une conduite agencée pour soumettre la partie restante (7) de gaz d'appoint à un refroidissement complet dans la section, en obtenant un flux principal de gaz d'appoint entièrement refroidi (9) à une température inférieure à celle du gaz d'appoint séparé (26) ;
d) une section de décalage eau-gaz (27) connectée à ladite conduite transportant la partie séparée du gaz d'appoint (26) et configurée pour produire un gaz décalé (28) enrichi en hydrogène ;
e) une section de refroidissement du gaz décalé et une première section de récupération d'hydrogène (33) agencée pour recevoir ledit gaz décalé après refroidissement et pour produire un premier flux d'hydrogène (34) ;
f) une conduite agencée pour ajouter ledit premier flux d'hydrogène (34) au flux principal de gaz d'appoint (9) et une conduite agencée pour ajouter un second flux d'hydrogène (20) obtenu à l'étape (i) audit gaz d'appoint, en obtenant ainsi un gaz d'appoint ajusté (11) ayant une teneur ajustée en hydrogène ;
g) une boucle de synthèse de méthanol (14) et une conduite agencée pour introduire le gaz d'appoint ajusté (11) dans ladite boucle dans laquelle la conversion catalytique des oxydes de carbone en méthanol est réalisée dans des conditions de synthèse de méthanol, en obtenant un méthanol brut condensé (15) ;
h) une section de purification du méthanol brut condensé (15), de préférence une section de distillation à multiples colonnes (49), obtenant du méthanol (56) ;
i) une seconde section de récupération d'hydrogène (19) agencée pour recevoir un flux de purge (16) extrait de la boucle de synthèse de méthanol et pour obtenir ledit second flux d'hydrogène (20) et un gaz résiduaire (21) contenant du méthane retiré du flux de purge ;
j) une conduite agencée pour introduire au moins une partie du gaz résiduaire (21) comme matière première dans la section de reformage pour la production du gaz d'appoint (3).

17. Usine selon la revendication 16, comportant une ou plusieurs caractéristiques suivantes :
la section de reformage comporte un reformeur autothermique, facultativement doté d'un ou plusieurs pré-reformeur(s) ;
la première section de récupération d'hydrogène est une unité PSA ;
la seconde section de récupération d'hydrogène est une unité de séparation à membrane ;
ladite section de décalage eau-gaz du gaz d'appoint séparé comporte un réacteur de décalage à haute température.

18. Installation selon la revendication 16 ou 17, dans laquelle la section de purification du méthanol brut condensé (15) de l'étape (h) comprend quatre colonnes fonctionnant en cascade, dans laquelle l'une des quatre colonnes est une colonne d'étêtage pour l'élimination de composants volatils, et les trois autres colonnes sont des colonnes de raffinage conçues pour séparer du méthanol de l'eau et des sous-produits d'alcool supérieur.
